(19) Europäisches Patentamt
European Patent Office
Office européen des brevets

(11) EP 2 153 736 B1

(12) EUROPEAN PATENT SPECIFICATION

(45) Date of publication and mention of the grant of the patent:
**19.04.2017 Bulletin 2017/16**

(21) Application number: **08752467.4**

(22) Date of filing: **08.05.2008**

(51) Int Cl.:
***A23L 33/115*** *(2016.01)* ***A23L 17/20*** *(2016.01)*

(86) International application number:
**PCT/JP2008/058581**

(87) International publication number:
**WO 2008/140026 (20.11.2008 Gazette 2008/47)**

# (54) NOVEL LEUKOTRIENE RECEPTOR ANTAGONIST FROM MARINE SOURCES

NEUER LEUKOTRIEN-REZEPTOR-ANTAGONIST AUS MEERESQUELLEN

ANTAGONISTE INÉDIT DU RÉCEPTEUR DES LEUCOTRIÈNES PROVENANT DES SOURCES MARINES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **11.05.2007 JP 2007127431**

(43) Date of publication of application:
**17.02.2010 Bulletin 2010/07**

(73) Proprietor: **Bizen Chemical Co., Ltd.**
**Okayama 709-0716 (JP)**

(72) Inventors:
- **KANADA, Teruyuki**
  **Wake-gun**
  **Okayama 709-0442 (JP)**
- **KUYAMA, Toru**
  **Okayama-shi**
  **Okayama 709-0635 (JP)**
- **HADA, Takahiko**
  **Okayama-shi**
  **Okayama 709-0856 (JP)**
- **ISHIHARA, Takafumi**
  **Akaiwa-shi**
  **Okayama 709-0716 (JP)**

(74) Representative: **Robson, Aidan John**
**Reddie & Grose LLP**
**16 Theobalds Road**
**London WC1X 8PL (GB)**

(56) References cited:

| | |
|---|---|
| **EP-A1- 1 027 833** | **WO-A1-92/21335** |
| **WO-A1-02/092540** | **WO-A1-2004/047554** |
| **WO-A1-2006/099591** | **WO-A1-2008/060163** |
| **WO-A2-03/011873** | **WO-A2-2006/111633** |
| **WO-A2-2008/149177** | **JP-A- 11 123 052** |
| **JP-A- 2000 239 168** | **JP-A- 2007 110 904** |
| **JP-A- 2007 145 814** | **US-A1- 2006 078 625** |

- **MAYSER P ET AL: "[omega]-3 Fatty acid-based lipid infusion in patients with chronic plaque psoriasis: Results of a double-blind, randomized, placebo-controlled, multicenter trial", JOURNAL OF THE AMERICAN ACADEMY OF DERMATOLOGY, C.V. MOSBY, ST. LOUIS, MO, US, vol. 38, no. 4, 1 January 1998 (1998-01-01), pages 539-547, XP002360544, ISSN: 0190-9622, DOI: DOI:10.1016/S0190-9622(98)70114-8**
- **KANEDA T. ET AL.: 'DHA wa Choju ni Kiyo Dekiruka Phospholipid to Triglyceride no Sa ni Tsuite' FOOD PROCESSING AND INGREDIENTS vol. 34, no. 8, 1999, pages 41 - 43, XP008122920**
- **INOUE Y. ET AL.: 'Manufacturing and Physiological properties of phosoholipids containing DHA' OLEOSCIENCE vol. 2, no. 2, 2002, pages 67 - 74, XP008122958**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 153 736 B1

## Description

### TECHNICAL FIELD

The present invention relates to a leukotriene receptor antagonist containing a phospholipid extract of a particular marine product.

### BACKGROUND ART

Leukotriene is a group of physiologically active substances synthesized in animal tissues from eicosapolyenoic acids such as arachidonic acid, and the substances are metabolic products of eicosanoids that exhibit physiological activity as transmitter substances against inflammation or allergic responses. Leukotrienes are structurally characterized by having oxygen at least at the C-5 position and having three conjugated double bonds, and there are six forms including type A to type F, and analogues thereof.

Each leukotriene has a corresponding receptor, but each of the leukotrienes and the leukotriene receptors are not necessarily in a one-to-one relationship. Furthermore, antagonists to the leukotriene receptors are known to be effective in the treatment and/or prevention of various diseases. For example, it is well known that the antagonists of leukotriene B4 receptors (BLT) have an effect of ameliorating ulcerative colitis, interstitial pneumonia, atopic dermatitis, contact-type dermatitis, psoriasis, bronchial asthma, chronic obstructive pulmonary diseases, sporadic lethal asthma and ischemic renal diseases. It is also well known that the antagonists of CysLT1 receptors, which are cysteinyl leukotriene receptors, have an effect of ameliorating bronchial asthma, bronchiolitis, allergic rhinitis, atopic dermatitis, mastocytoma and ischemic cardiac diseases.

In regard to the antagonists of these receptors, there are present known substances, but these substances are expensive, and production thereof is difficult. Furthermore, as for certain substances, there is a fear for their toxicity. Thus, there is a demand for a leukotriene receptor antagonist that can be simply prepared from a natural source.

The following are the prior art literature data related to the invention of the present application.

Nonpatent Literature 1: BLT1 and BLT2: The Leukotriene B4 Receptors. Tager AM, Luster AD, Prostaglandins Leukot Essent Fatty Acid. 2003 Aug-Sep 69(2-3) 123-34

Nonpatent Literature 2: Cysteinyl-Leukotriene receptor antagonists: Present Situation and Future Opportunities. Capra V, Ambrosio M, Riccioni G, Rovati GE, Curr Med Chem. 2006 13(26) 3213-26

Nonpatent Literature 3: Leukotriene Lipoxygenase: Metabolic System and Drug Discovery, Masayoshi Abe, Tanihiro Yoshimoto, Folia Pharmacologica Japonica 124, 415-425 (2004)

WO 2004/047554 discloses the use of certain marine phospholipids to treat inflammatory bowel disease or inflammatory skin diseases.

### DISCLOSURE OF INVENTION

The present invention aims to provide a leukotriene receptor antagonist that can be conveniently produced from a natural source.

The inventors found that a phospholipid extract of a marine product contains antagonists of BLT and CysLT1, which are leukotriene receptors.

The present invention provides a composition for use in treating or preventing a disease selected from ulcerative colitis, allergic rhinitis and mastocytoma by inhibiting the binding of a leukotriene to a leukotriene receptor, comprising a phospholipid extract of a marine product selected from squid and Akiami paste shrimp.

The phospholipid extract of a marine product is preferably an ethanol extract.

The leukotriene receptor is preferably a BLT receptor.

The leukotriene receptor is preferably a CysLT1 receptor.

The composition may be a dietary composition.

The composition may be a pharmaceutical composition.

### BEST MODE(S) FOR CARRYING OUT THE INVENTION

Hereinafter, the present invention will be described. Throughout the present specification, unless particularly stated otherwise, a singular expression should be definitely understood to include the concept of plurality as well. Furthermore, the terms used in the present specification should be definitely understood to have the meanings conventionally used in the pertinent field.

Hereinafter, the definitions of the terms used particularly in the present invention will be listed.

As used herein, the term "leukotriene" refers to a group of physiologically active substances synthesized in animal tissues from eicosapolyenoic acids such as arachidonic acid, which substances are metabolic products of eicosanoids that exhibit physiological activity as transmitter substances for inflammation or allergic responses.

As used herein, the term "leukotriene B4" is used interchangeably with "$LTB_4$" and refers to a compound having the following structural formula:

[Formula 1]

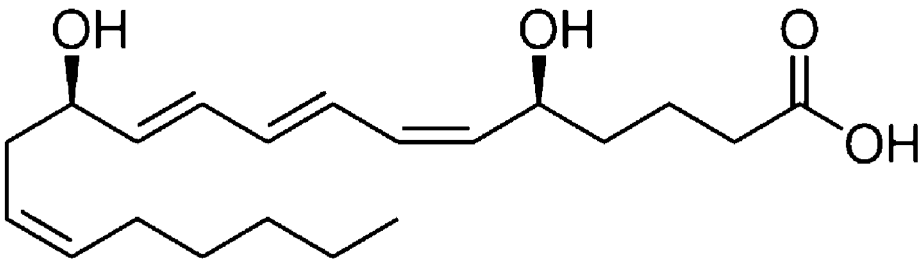

As used herein, the term "leukotriene C4" is used interchangeably with "$LTC_4$" and refers to a compound having the following structural formula:

[Formula 2]

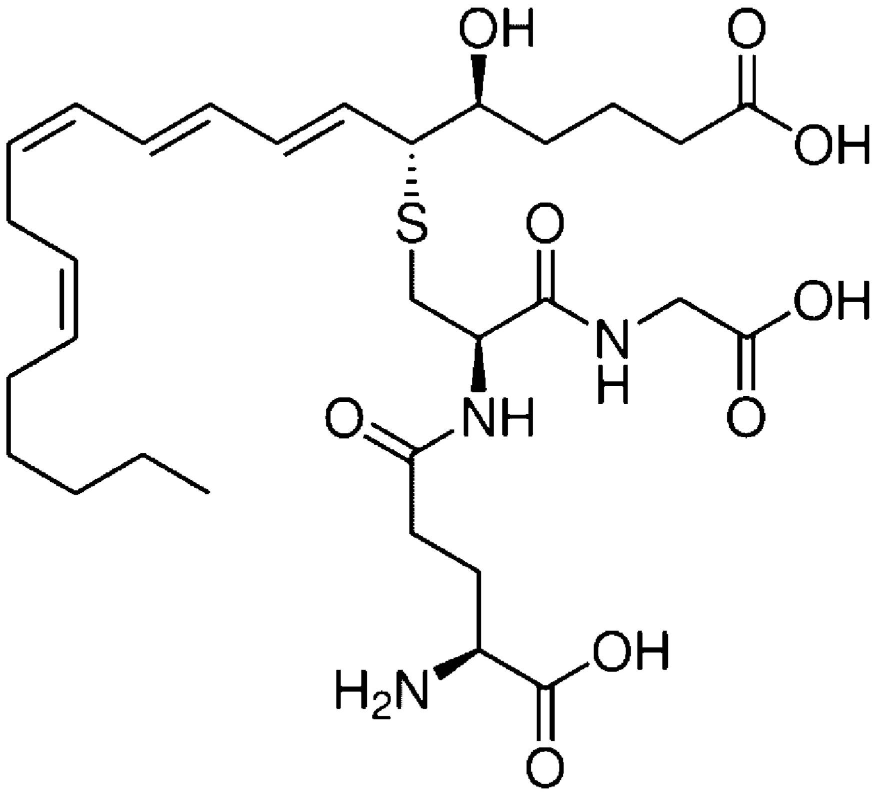

As used herein, the term "leukotriene D4" is used interchangeably with "$LTD_4$" and refers to a compound having the following structural formula:

[Formula 3]

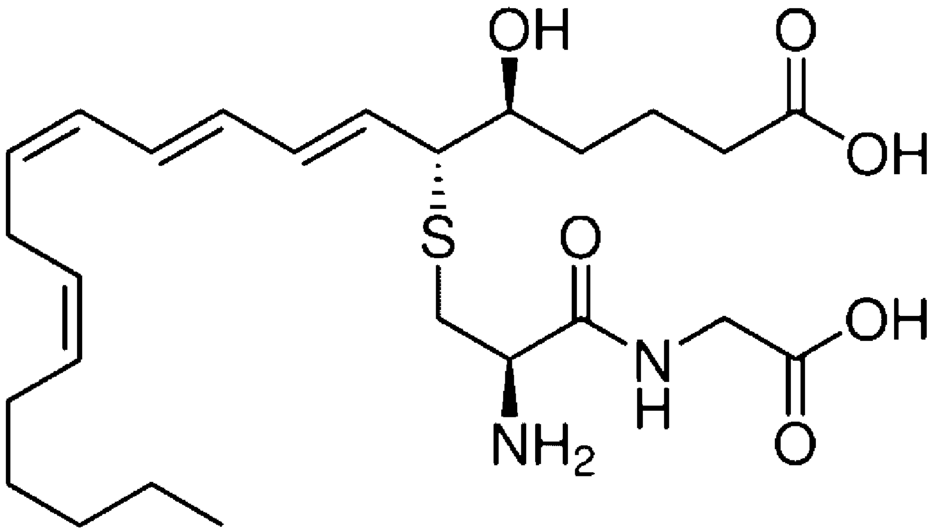

As used herein, the term "leukotriene E4" is used interchangeably with "$LTE_4$" and refers to a compound having the following structural formula:

[Formula 4]

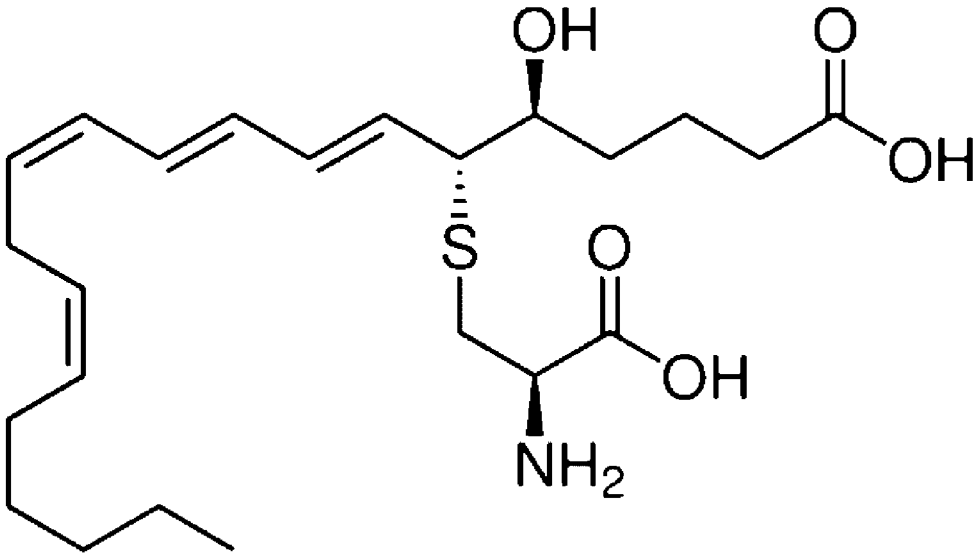

As used herein, the term "cysteinyl leukotriene" refers to a leukotriene containing cysteine, which is an amino acid, and examples thereof include, but not limited to, leukotriene C4, leukotriene D4 and leukotriene E4.
As used herein, the term "CysLT1 receptor" refers to a receptor to which a cysteinyl leukotriene binds.
As used herein, the "phospholipid extract" refers to a substance that is extracted from a predetermined source and contains phospholipids.
As used herein, the "phospholipids" include glycerophospholipids and sphingophospholipids.
As used herein, the term "sphingophospholipids" refers to sphingolipids containing phosphorus. As used herein, the term "sphingolipids" refers to lipids having long chain bases, such as sphingosine. Examples of the sphingolipids include, but not limited to, sphingosine.
As used herein, the term "glycerophospholipids" refers to glycerophosphoric acid and derivatives thereof. Glycerophosphoric acid exists as isomers, and includes sn-glycerol-1-phosphoric acid, sn-glycerol-2-phosphoric acid and sn-glycerol-3-phosphoric acid. In the living body, sn-glycerol-3-phosphoric acid (that is, L-glycerol-3-phosphoric acid, D-glycerol-1-phosphoric acid and L-$\alpha$-glycerophosphoric acid) is available. Examples of the derivatives of glycerophosphoric acid that fall under the definition of the glycerophospholipids according to the present specification include phosphatidylcholine, phosphatidylethanolamine, phosphatidylinositol and phosphatidylserine, which have fatty acids bonded to the 1-position and the 2-position and have choline, ethanolamine, inositol, serine and the like bonded to the phosphoric acid group of glycerophosphoric acid, as well as substances having phosphatidic acid bonded thereto, but are not limited to these substances. Lysophospholipids of these substances are also included in the glycerophospholipids as the derivatives of glycerophosphoric acid of the present invention.

As for the source of the phospholipids that are used in the present invention, marine products squid and Akiami paste shrimp are used. Preferable examples of squid include squid tissues of swordtip squid, golden cuttlefish, Pacific flying squid, Japanese flying squid, Japanese common squid, firefly squid, spear squid and the like, and skins thereof, (for example, edible parts), but are not limited to these. The oil component obtainable from the squid tissues is a preferable source of the phospholipids of the present invention. The squid skin (mantle muscle) is a preferable source of the phospholipids of the present invention.

As the method of extracting the "phospholipid extract" of the present invention, for example, known methods such as a pressing method, a boiling method and a solvent extraction method, can be used. In the pressing method, as a pre-treatment, any foreign substances are excluded from the raw material, and the raw material is pulverized to an appropriate size and then heated. As a result of heating, effects such as (1) disruption of the cellular membrane, (2) coagulation of proteins, and (3) a decrease in the viscosity of oils and fats are obtained. When the heated raw material is placed in a press, and pressure is applied, oil contents are squeezed out. Here, the obtained oil contents are commonly referred to as crude oil and contain glycerides as the main component. Therefore, when water is added to these oil contents to perform a degumming treatment, and the separated gum substance portion is dried, a phospholipid-containing fraction having a phospholipids content of 20 to 60% by weight can be obtained. The boiling method is usually a method of adding water to the raw material, heating the raw material to boil, and then separating floating oil contents from an aqueous layer to obtain the oil contents. Here, the obtained oil contents are commonly referred to as crude oil and contain glycerides as the main component. Therefore, when water is added to these oil contents to perform a degumming treatment, and the separated gum substance portion is dried, a phospholipid-containing fraction having a phospholipids content of 20 to 60% by weight can be obtained.

In the case of the solvent extraction method, the process can be carried out by extracting active ingredients with a solvent, without performing any pre-treatment on the source, or after freeze-drying the source. Examples of the solvent include an oil-soluble organic solvent, a mixed solvent of an oil-soluble organic solvent and a hydrophilic alcohol, and a hydrophilic alcohol-containing solvent, but are not limited to these. Examples of the oil-soluble organic solvent include, but not limited to, acetone, hexane, ether and petroleum ether, and combinations thereof. Examples of the hydrophilic alcohol include, but are not limited to, methanol, ethanol, isopropanol and butanol, and combinations thereof.

Solvent extraction is performed by extracting a fraction of oil contents at normal temperature or under heating, at normal pressure or under pressure, with stirring as necessary. This extracted oil component fraction is commonly referred to as crude oil and contain glycerides as the main component. Therefore, when water is added to these oil contents to perform a degumming treatment, and the separated gum substance portion is dried, a phospholipid-containing fraction having a phospholipids content of 20 to 60% by weight can be obtained. Alternatively, if extraction is carried out using a hydrated solvent of the above-described hydrophilic alcohol, the same phospholipid-containing oil contents as described above is obtained as the extract.

In obtaining such a fractionation product of oil contents, a liquid-liquid separation method utilizing the difference in the solubility of the active ingredient in different solvents, is convenient and practical. For example, a fractionation product having a further increased phospholipid content can be obtained by performing an acetone precipitation treatment on an extract that has been prepared by using ethanol. Furthermore, a fractionation product having a phospholipid content of 90% by weight or more can also be obtained by performing a chromatography treatment on an ethanol extract, using a column packed with an adsorbent such as silica gel or alumina.

According to a desirable embodiment, the phospholipid-containing oil contents thus obtained and a fractionation product thereof has a phospholipid content of 20% by weight or more, and preferably 30% by weight or more. In the phospholipids prepared by the method described in the following examples, since highly unsaturated fatty acids are contained as constituent fatty acids of the phospholipids, the phospholipids have excellent stability against oxidation as compared with highly unsaturated fatty acid triglycerides of fish oil or the like, and thus, the phospholipids hardly have any odor returning after purification or unpleasant odor.

The pharmaceutical composition and dietary composition of the present invention contain the phospholipids or oil contents containing the phospholipids, as an active ingredient. The pharmaceutical composition and dietary composition of the present invention desirably have a phospholipid content of 20% by weight or more, and more preferably 30% by weight or more. The compositions may also contain other known components or raw materials appropriately in

combination, as long as within the scope and extent of not impairing the desired effects of the present invention. Examples of thereof include, but are not limited to, ascorbic acid, amino acids, peptides, proteins and degradation products thereof, various sugars, starches and degradation products thereof, minerals, vitamin E, tocopherol, phytosterols, catechin, polyphenols of guava leaves and the like, and derivatives thereof.

If these substances for combination are oil-soluble such as in the case of ascorbic acid palmitate, phytosterols, vitamin E and the like, these substances are mixed with the phospholipids according to the present invention or the oil contents containing the phospholipids to form a uniform state. If the substances for combination are water-soluble or water-dispersible such as in the case of ascorbic acid, amino acids, minerals, proteins and the like, for example, dried powders thereof are kneaded together with the phospholipids according to the present invention or the oil contents containing the phospholipids to form a dispersed state, or water and surfactants as appropriate are incorporated to form an emulsified state.

According to the present invention, as described previously, there is provided a dietary composition/pharmaceutical composition containing a phospholipid extract of a marine product selected from squid and Akiami paste shrimp, as an active ingredient, said composition being for the treatment as set out in the claims.

(Prescription of pharmaceutical composition)

There is disclosed a method for the remedy and/or prevention of a disease or a disorder set out in the claims by administering an effective amount of a therapeutic agent to a test subject. The therapeutic agent means the composition of the present invention, which is combined with a pharmaceutically acceptable carrier form (for example, a sterilized carrier).

The therapeutic agent is prescribed and administered in a manner adhering to the Good Medical Practice (GMP), while taking into consideration of the clinical conditions of individual patients (particularly, adverse side effects caused by a remedy using the therapeutic agent alone), the site of delivery, method of administration, regime of administration, and other factors that are known to those skilled in the art. Therefore, the aimed "effective amount" is determined on the basis of such consideration in the present specification.

As a general suggestion, the total pharmaceutically effective amount of the therapeutic agent that is orally administered per dose is in the range of about 2 μg/kg/day to 1000 mg/kg/day based on the patient's body weight, but as described above, this amount is left to the physician's discretion. More preferably, this dose is at least 10 mg/kg/day, and most preferably between about 20 mg/kg/day and about 1000 mg/kg/day for a person. As another general suggestion, the total pharmaceutically effective amount of the therapeutic agent that is parenterally administered per dose is in the range of about 0.2 μg/kg/day to 250 mg/kg/day based on the patient's body weight, but as described above, this amount is left to the physician's discretion. More preferably, this dose is at least 1 mg/kg/day, and most preferably between about 5 mg/kg/day and about 25 mg/kg/day for a person.

The therapeutic agent may be administered orally, intrarectally, parenterally, intracisternally, intravaginally, intraperitoneally, locally (by means of a powder, an ointment, a gel, a drip infusion, or a transdermal patch), or as an intrabuccal, peroral or intranasal spray. The representative route of administration of the composition of the present invention is oral administration.

The "pharmaceutically acceptable carrier" refers to a non-toxic, solid, semi-solid or liquid filler, diluent or encapsulating material, or a prescribed auxiliary agent of any form.

The composition of the present invention is also appropriately administered by a sustained release system. An appropriate example of the sustained release therapeutic agent may be administered orally, intrarectally, parenterally, intracisternally, intravaginally, intraperitoneally, locally (by means of a powder, an ointment, a gel, a drip infusion, or a transdermal patch), or as an intrabuccal, peroral or intranasal spray. A "pharmaceutically acceptable carrier" refers to a non-toxic, solid, semi-solid or liquid filler, diluent or encapsulating material, or a prescribed auxiliary agent of any form. The term "parenterally" as used herein means a form of administration including intravenous, intramuscular, intraperitoneal, intrasternal, subcutaneous and intraarterial injection and infusion.

The composition of the present invention is also appropriately administered by a sustained release system. Appropriate examples of the sustained release therapeutic agent include an appropriate polymer material (for example, a semi-permeable polymer matrix in the form of a molded article (for example, a film or a microcapsule)), an appropriate hydrophobic material (for example, as an emulsion in an oil of acceptable quality), or an ion-exchanged resin, and a poorly soluble derivative (for example, a poorly soluble salt).

Examples of the sustained release matrix include polylactide (USP 3,773,919, EP 58,481), a copolymer of L-glutamic acid and γ-ethyl-L-glutamate (Sidman, et al., Biopolymers 22:547-556 (1983)), poly(2-hydroxyethyl methacrylate) (Langer, et al., J. Biomed. Mater. Res. 15: 167-277 (1981), and Langer, Chem. Tech. 12:98-105 (1982)), ethylene vinyl acetate (Langer, et al., ibid) or poly-D-(-)-3-hydroxybutyric acid (EP 133,988).

The sustained release therapeutic agent also includes the therapeutic agent that is encapsulated in liposomes (generally see Langer, Science 249:1527-1533 (1990); and Treat, et al., Liposomes in the Therapy of Infectious Disease

and Cancer, Lopez-Berenstein and Fidler (edited), Liss, New York, pp. 317-327 and 353-365 (1989)). The liposomes containing the therapeutic agent may be prepared by methods that are known per se: DE 3,218,121; Epstein, et al., Proc. Natl. Acad. Sci. USA 82:3688-3692 (1985); Hwang et al., Proc. Natl. Acad. Sci. USA 77: 4030-4034 (1980); EP 52,322; EP 36,676; EP 88,046; EP 143,949; EP 142,641; Japanese Patent Application No. 83-118008; USP 4,485,045 and USP 4,544,545; and EP 102,324. Generally, liposomes are small (about 200 to 800 A) unilamellar type, and the lipid content therein is even greater than the content of cholesterol by about 30% by mol. The selected proportions are adjusted for optimal therapeutic agents.

Alternatively, the composition of the present invention may be delivered by a pump (see Langer, ibid; Sefton, CRC Crit. Ref. Biomed. Eng. 14:201 (1987); Buchwald, et al., Surgery 88: 507 (1980); and Saudek, et al., N. Engl. J. Med. 321: 574 (1989)).

Other controlled release systems are discussed in the review written by Langer (Science 249: 1527-1533 (1990)).

For the parenteral administration, the therapeutic agent is mixed, at a purity at a desired degree, with a pharmaceutically acceptable carrier, that is, a substance that is non-toxic to the receiver at the dosage and concentration of use, and is compatible with other components of the prescription, in an injectable form of a unit dosage (solution, suspension or emulsion). For example, this prescription preferably does not include any other compound that is known to undergo oxidation and to be harmful to the therapeutic agent.

In general, the prescription is prepared by uniformly and intimately contacting the therapeutic agent with a liquid carrier or a finely divided solid carrier, or with both of the carriers. Subsequently, if necessary, the product is formed into a desired prescription. Preferably, the carrier is a parenteral carrier, and more preferably a solution that is isotonic with the blood of the receiver. Examples of such a carrier vehicle include water, physiological saline, Ringer's solution and a dextrose solution. Non-aqueous vehicles such as non-volatile oils and ethyl oleate are also useful herein as in the case of the liposomes.

The carrier appropriately contains a trade amount of additives such as a substance that enhance isotonicity and chemical stability. Such a substance is non-toxic to the receiver at the dosage and concentration of use, and examples of such a substance include buffering agents such as phosphates, citrates, succinates, acetates and other organic acids or salts thereof; antioxidants such as ascorbic acid; low molecular weight (fewer than about 10 residues) polypeptides (for example, polyarginine or tripeptides); proteins such as serum albumin, gelatin or immunoglobulins; hydrophilic polymers such as polyvinylpyrrolidone; amino acids such as glycine, glutamic acid, aspartic acid and arginine; monosaccharides, disaccharides and other carbohydrates, including cellulose or derivatives thereof, glucose, mannose or dextrin; chelating agents such as EDTA; sugar alcohols such as mannitol or sorbitol; counterions such as sodium; and/or nonionic surfactants such as polysorbate, poloxamer or PEG.

The therapeutic agent is prescribed in such a vehicle representatively at a concentration of about 10 mg/ml to 1000 mg/ml, and preferably 50 to 1000 mg/ml, at about pH 6 to 9. It is understood that salt is formed by using the specific excipient, carrier or stabilizer mentioned above.

Any drug that is to be used in the therapeutic administration may be in a state not containing any organism/virus other than the virus as an active ingredient, that is, in a sterile state. The sterile state is easily achieved by filtering a drug through a sterilized filter membrane (for example, 0.2-micrometer membrane). In general, the therapeutic agent is placed in a container having a sterilized access port, for example, a solution bag or vial for intravenous use, having a stopper that can be punctured with a subcutaneous injection needle.

The therapeutic agent is conventionally stored as an aqueous solution or a freeze-dried prescription for reconstitution, in a unit dose or multidose container, for example, in a sealed ampoule or vial. As an example of the freeze-dried prescription, 5 ml of a sterile filtered 5% (w/v) aqueous solution of the therapeutic agent is filled in a 10-ml vial, and the obtainable mixture is freeze-dried. The freeze-dried therapeutic agent is reconstituted using bacteriostatic water for injection, to prepare an infusion solution.

The disclosure also provides a pharmaceutical pack or kit including one or more containers filled with one or more components of the composition of the present invention. An indication that regulates the production, use or sale of a pharmaceutical product or biological product, written in the format provided by a government agency, may be attached to such a container, and this indication shows the approval made by the government agency in relation to the production, use or sale of the administration to human being. Furthermore, the therapeutic agent can also be used in combination with other therapeutic compounds.

The composition of the present invention may be administered alone or in combination with other therapeutic agents. The combination may be administered, for example, simultaneously as a mixture; simultaneously or concurrently, but separately; or over time. This includes the suggestion that the combined drugs are administered together as a therapeutic mixture, and the protocol in which the combined drugs are administered separately but simultaneously, for example, to the same individual through different venous lines. The administration "in combination" further includes separate administration of one compound or drug that is given firstly, and then secondly.

In regard to the formulation of the phospholipid extract of a marine product of the present invention, the previously

described extract may be directly used, but this may also be further fractionated and used as a fractionation product containing the active ingredient phospholipids at higher concentration. In the case of purifying the phospholipid extract used in the present invention, the purification can be carried out by taking the extent of the content of docosahexaenoic acid (DHA) in a fraction containing phospholipids, as an indicator.

(Preparation of dietary composition)

A suitable embodiment of the present invention is a dietary composition. That is, a composition containing the phospholipids that are obtained as described previously as an active ingredient, can be directly added to foods in the liquid state, gel state or solid state, for example, juice, soft drinks, coffee, black tea, green tea, oolong tea, vegetable juice, natural fruit juice, milk beverages, milk, soy milk, sports beverages, near-water beverages, nutritional supplement beverages, coffee beverages, hot chocolate, soup, dressing, mousse, jelly, yogurt, pudding, rice topping (furikake), powdered milk for infants, processed milk, sports drinks, nutritional drinks, cake mixes, bread, pizza, pies, crackers, biscuits, cakes, cookies, spaghetti, macaroni, pasta, Japanese wheat noodle (udon), soba noodle, chinese noodle, candies, toffees, chewing gums, chocolate, sticky rice biscuits, potato chips, snacks, ice cream, sherbet, cream, cheese, powdery or liquid dairy products such as powdered milk, condensed milk and milk beverages, steamed bean-jam bun, uiro, mochi, rice cakes, soy sauce, mop sauce, noodle sauce, sauces, instant bouillon, stew sauce, soup stock, seasoning, curry sauce, mayonnaise, ketchup, retort curry sauce, retort stew, retort soup, retort rice bowl, canned food, ham, hamburger, meatball, croquette, dumpling, pilaf, rice ball, frozen meals and refrigerated meals, fish sausage, fish ball, rice for box lunch, sushi, milk for infants, weaning food, baby food, sports foods, nutritional supplement foods, supplements, health foods and the like. If necessary, the pharmaceutical composition or dietary composition can also be processed into pellets, tablets, granules or the like, together with excipients such as dextrin, lactose and starch, or with fragrances, colorants and the like, or coated with gelatin or the like and formed into capsules, and can be used as health foods, nutritional supplement foods or the like. In regard to the mixing amount of the squid phospholipid extract in these food products or dietary compositions, it is difficult to define the amount collectively due to the different type, state or the like of the food product of composition, but the amount is about 0.01 to 50% by weight, and preferably 0.1 to 30% by weight. If the mixing amount is less than 0.01% by weight, the desired effect obtainable by oral ingestion is insignificant, and if the mixing amount is greater than 50% by weight, the flavor may be impaired depending on the type of food product, or the food product may not be prepared in some cases. The phospholipid extract of a marine product used in the present invention can be directly provided for diet without any problem.

The composition of the present invention is used as a dietary composition/pharmaceutical composition for inhibiting the activity of leukotriene receptors. Examples of the leukotriene receptors whose activity is to be inhibited, include BLT receptors and CysLT1 receptors, but are not limited to these. Such a composition having an inhibitory activity against BLT receptors is useful as a composition for the prevention and/or treatment of a disease selected from ulcerative colitis, interstitial pneumonia, atopic dermatitis, contact-type dermatitis, psoriasis, bronchial asthma, chronic obstructive pulmonary diseases, sporadic lethal asthma, and ischemic renal diseases. Such a composition having an inhibitory activity against CysLT1 receptors is useful as a composition for the prevention and/or treatment of a disease selected from the group consisting of bronchial asthma, bronchiolitis, allergic rhinitis, atopic dermatitis, mastocytoma, and ischemic cardiac diseases.

There is disclosed a method of using a phospholipid extract of a marine product for the manufacture of these compositions and/or medicines. A method for inhibiting the activity of leukotriene receptors includes a step of administering a phospholipid extract of a marine product.

Hereinafter, the present invention will be described in detail by way of examples and the like, but the present invention is not intended to be limited to these examples.

[EXAMPLES]

(Example 1)

Japanese common squids landed at Sakaiminato were removed of the internal organs and were washed with water. Then, the squids were cut and dehydrated by freeze-drying. 10 kg of this dried Japanese common squids was added with 20 L of 99% (V/V) ethanol, and the mixture was subjected to extraction by appropriately mildly stirring for 2 hours at 60°C. The mixture was filtered to obtain an extract, and then 20 L of 99% (V/V) ethanol was added to the residue to perform extraction in the same manner. The mixture was filtered to obtain an extract, and this extract was combined with the first extract. Ethanol was distilled off under reduced pressure from the extract, and thus 2.5 kg of a paste-like extract. The entire amount of this extract was completely dissolved in a mixed liquid of 8 L of chloroform and 4 L of methanol, and then 3 L of water was added to the solution. The mixture was adequately stirred, and after stopping the stirring, the mixture was left to stand still. When the solution separated into two layers, the lower chloroform layer was

recovered. Chloroform was distilled off under reduced pressure, to obtain a paste-like purification product. The purification product was analyzed using high performance liquid chromatography (HPLC), gas chromatography (GLC) and thin layer chromatography (TLC), and as a result, the purification product was found to contain the following: glycerophospholipid content: 75.6% by weight (specifically, phosphatidylcholine: 58.2%, lysophosphatidylcholine: 5.2%, phosphatidylethanolamine: 26.1 %, lysophosphatidylethanolamine: 2.7%, phosphatidylinositol: 1.5%, others: 6.3%); within the total fatty acid composition, DHA content: 40.3% by weight, EPA content: 15.6% by weight; among glycerophospholipids, DHA content: 41.5% by weight, and EPA content: 16.2% by weight.

(Example 2)

Frozen Akiami paste shrimps were purchased, and the shrimps were dehydrated by freeze-drying. 10 kg of this dried Akiami paste shrimps was added with 20 L of 99% (V/V) ethanol, and the mixture was subjected to extraction by appropriately mildly stirring for 2 hours at 60°C. The mixture was filtered to obtain an extract, and then 20 L of 99% (V/V) ethanol was added to the residue to perform extraction in the same manner. The mixture was filtered to obtain an extract, and this extract was combined with the first extract. Ethanol was distilled off under reduced pressure from the extract, and thus 2.1 kg of an oily extract. The entire amount of this extract was added to 20 L of cold acetone, and the mixture was appropriately stirred. After stopping the stirring, the mixture was left to stand still. The acetone solution was removed by decantation, and the precipitate was recovered. The recovered precipitate was subjected to distillation under reduced pressure, and thus a paste-like concentrate was obtained. The entire amount of this concentrate was completely dissolved in a mixed liquid of 8 L of chloroform and 4 L of methanol, and then 3 L of water was added to the solution. The mixture was adequately stirred, and after stopping the stirring, the mixture was left to stand still. When the solution separated into two layers, the lower chloroform layer was recovered. Chloroform was distilled off under reduced pressure, to obtain a paste-like purification product. The purification product was analyzed using high performance liquid chromatography (HPLC), gas chromatography (GLC) and thin layer chromatography (TLC), and as a result, the purification product was found to contain the following: glycerophospholipid content: 78.8% by weight (specifically, phosphatidylcholine: 80.2%, lysophosphatidylcholine: 2.2%, phosphatidylethanolamine: 5.3%, phosphatidylinositol: 1.5%, phosphatidylserine: 1.2%, others: 9.6%); within the total fatty acid composition, DHA content: 10.3% by weight, EPA content: 15.8% by weight; among glycerophospholipids, DHA content: 12.5% by weight, and EPA content: 18.3% by weight.

(Example 3: Measurement of antagonist activity against CysLT1 receptors)

The antagonist activities of the squid extract and Akiami paste shrimp extract used in the present invention against leukotriene receptor CysLT1 were measured, as well as, by way of reference, krill extract.

(Measurement of CysLT1 receptor binding capability)

The CysLT1 receptor binding capability of a squid extract, an Akiami paste shrimp extract and a krill extract was measured by a conventional method using genetically modified Chinese hamster ovary (CHO-K1) cells. Specifically, a substitution reaction between 0.3 nM $^3$H-labeled $LTD_4$ and phospholipids (200 $\mu$g/ml) was performed at 25°C for 30 minutes, and the substitution reaction was measured based on the radioactivity. The squid phospholipids prepared in Example 1 as the squid extract, the Akiami paste shrimp phospholipids, and the krill phospholipids, and soybean phospholipids as a control ((Tsuji Oil Mill Co., Ltd.) 565-1 Ureshino Niwanoshocho, Matsusaka-shi, Mie-ken, 515-2314) were used. The amount of $^3$H-labeled $LTD_4$ binding in the instance where no sample was added, was designated as the total binding amount, and the amount of $^3$H-labeled $LTD_4$ binding in the instance where a sample was added, was designated as the sample binding amount. The inhibitory rate was determined by the following calculation formula:

$$\text{Inhibitory rate} = (\text{total binding amount} - \text{binding amount upon sample addition})/\text{specific binding amount} \times 100$$

Here, the specific binding amount was 93%.

As a result, while the inhibitory activity of the soybean phospholipids of the control was only 63%, the inhibitory activity of the squid phospholipids prepared in Example 1 was 91%, and the inhibitory activity of the Akiami paste shrimp phospholipids prepared in Example 2 was 94%. From these results, it was demonstrated that the squid extract and Akiami paste shrimp extract used in the present invention contain antagonists that inhibit the binding of leukotrienes to CysLT1 receptors.

[Table 1]

| Activity of CysLT1 receptor binding capability | |
|---|---|
| Sample concentration (200 μg/ml) | CysLT1 receptor binding capability (% substitution) |
| Squid phospholipids | 91 |
| Akiami paste shrimp phospholipids | 94 |
| Krill phospholipids | 58 |
| Soybean phospholipids | 63 |

(Measurement of antagonist activity against CysLT1 receptors)

The antagonist activity of phospholipids against CysLT1 receptors was measured by a conventional method using the ileum of a Guinea pig. Specifically, the ileum of a Guinea pig was extracted and incubated in Krebs Buffer (Krebs liquid: $KH_2PO_4$ 1.2 mM, NaCl 120.0 mM, KCl 5.0 mM, $MgSO_4$ 1.2 mM, $NaHCO_3$ 25.0 mM, EDTA-2Na 0.027 mM, $CaCl_2$ 2.4 mM, and glucose 11.0 mM (pH 7.4)) for 5 minutes at 32°C. Subsequently, 10 nM $LTD_4$ was added thereto, and the degree of contraction (the values obtained by measuring the length of contraction for 5 minutes when squid phospholipids were added and when no phospholipids were added) was measured for 5 minutes. A comparison was made between the instance where squid phospholipids were added at a concentration of 500 μg/ml and the instance where no phospholipids were added, and the antagonist activity of the squid phospholipids was determined. As a result, it was confirmed that the squid extract used in the present invention actually has an antagonist activity. The antagonist activity was 31%.

(Example 4: Measurement of antagonist activity against BLT receptors)

The antagonist activities of the squid extract and Akiami paste shrimp extract used in the present invention against leukotriene receptor BLT were measured, as well as, by way of reference example, krill extract.

(Measurement of BLT receptor binding capability)

The activity of the BLT receptor binding capability of phospholipids was measured by a conventional method using human leukemia-derived cells (U937). Specifically, a substitution reaction between 0.2 M $^3$H-labeled $LTB_4$ and phospholipids (200 μg/ml) was performed at 25°C for 30 minutes, and the substitution reaction was measured based on the radioactivity. As for the phospholipids, the phospholipids prepared in Example 1 and Example 2, and soybean phospholipids as a control were used in the same manner as in Example 3. The amount of $^3$H-labeled $LTB_4$ binding in the instance where no sample was added, was designated as the total binding amount, and the amount of $^3$H-labeled $LTB_4$ binding in the instance where a sample was added, was designated as the sample binding amount. The inhibitory rate was determined by the following calculation formula:

$$\text{Inhibitory rate} = (\text{total binding amount - binding amount upon sample addition})/\text{specific binding amount} \times 100$$

Here, the specific binding amount was 85%.

As a result, while the inhibitory activity of the soybean phospholipids of the control was only 8%, the inhibitory activity of the squid phospholipids prepared in Example 1 was 40%, and the inhibitory activity of the Akiami paste shrimp phospholipids prepared in Example 2 was 79%, which values were very high. From these results, it was demonstrated that the squid extract and Akiami paste shrimp extract used in the present invention contain antagonists that strongly inhibit the binding of leukotrienes to BLT receptors. It was also demonstrated that krill phospholipids also contain antagonists that inhibit the binding of leukotrienes to BLT receptors.

[Table 2]

| Activity of BLT receptor binding capability | |
|---|---|
| Sample concentration (200 μg/ml) | BLT receptor binding capability (% substitution) |
| Squid phospholipids | 40 |
| Akiami paste shrimp phospholipids | 79 |
| Krill phospholipids | 21 |
| Soybean phospholipids | 8 |

As discussed above, the present invention has been illustrated using preferred embodiments of the present invention, but the present invention is not to be interpreted to be limited to these embodiments. It should be understood that the scope of the present invention is definitely interpreted only on the basis of the scope of the claims. It should be understood that a person ordinarily skilled in the art can carry out an equivalent scope of invention based on the descriptions of the present invention and the common technological knowledge, by referring to the descriptions of the specific preferred embodiments of the present invention.

INDUSTRIAL APPLICABILITY

Antagonists of leukotriene receptors that are believed to have various pharmacological activities are conveniently provided from natural sources. The compositions of the present invention are useful as pharmaceutical compositions and dietary compositions, as claimed.

**Claims**

1. A composition, for use in treating or preventing a disease selected from ulcerative colitis, allergic rhinitis, and mastocytoma by inhibiting the binding of a leukotriene to a leukotriene receptor, comprising a phospholipid extract of a marine product selected from squid and Akiami paste shrimp.

2. The composition for use in treating or preventing the diseases listed according to claim 1, wherein the phospholipid extract of a marine product is an ethanol extract.

3. The composition for use in treating or preventing the diseases listed according to claim 1, wherein the leukotriene receptor is a BLT receptor.

4. The composition for use in treating or preventing the diseases listed according to claim 1, wherein the leukotriene receptor is a CysLT1 receptor.

5. The composition for use in treating or preventing the diseases listed according to any one of claims 1-4, wherein the composition is a dietary composition.

6. The composition for use in treating or preventing the diseases listed according to any one of claims 1-4, wherein the composition is a pharmaceutical composition.

**Patentansprüche**

1. Zusammensetzung zur Verwendung bei der Behandlung oder Verhütung einer Krankheit ausgewählt aus Colitis ulcerosa, allergischer Rhinitis und Mastozytom, durch Inhibieren der Bindung eines Leukotriens an einen Leukotrienrezeptor, die einen Phospholipidextrakt eines Meeresprodukts beinhaltet, das aus Tintenfisch und Akiami-Garnele ausgewählt ist.

2. Zusammensetzung zur Verwendung bei der Behandlung oder Verhütung der aufgelisteten Krankheiten nach Anspruch 1, wobei der Phospholipidextrakt eines Meeresprodukts ein Ethanolextrakt ist.

**3.** Zusammensetzung zur Verwendung bei der Behandlung oder Verhütung der aufgelisteten Krankheiten nach Anspruch 1, wobei der Leukotrienrezeptor ein BLT-Rezeptor ist.

**4.** Zusammensetzung zur Verwendung bei der Behandlung oder Verhütung der aufgelisteten Krankheiten nach Anspruch 1, wobei der Leukotrienrezeptor ein CysLT1-Rezeptor ist.

**5.** Zusammensetzung zur Verwendung bei der Behandlung oder Verhütung der aufgelisteten Krankheiten nach einem der Ansprüche 1-4, wobei die Zusammensetzung eine diätetische Zusammensetzung ist.

**6.** Zusammensetzung zur Verwendung bei der Behandlung oder Verhütung der aufgelisteten Krankheiten nach einem der Ansprüche 1-4, wobei die Zusammensetzung eine pharmazeutische Zusammensetzung ist.

## Revendications

**1.** Composition, destinée à être utilisée dans le traitement ou la prévention d'une maladie sélectionnée parmi la colite ulcéreuse, la rhinite allergique, et le mastocytome en inhibant la liaison d'un leucotriène à un récepteur de leucotriène, comprenant un extrait de phospholipides d'un produit marin sélectionné parmi le calmar et la crevette Akiami.

**2.** Composition destinée à être utilisée dans le traitement ou la prévention des maladies citées selon la revendication 1, dans lequel l'extrait de phospholipides d'un produit marin est un extrait d'éthanol.

**3.** Composition destinée à être utilisée dans le traitement ou la prévention des maladies citées selon la revendication 1, dans lequel le récepteur de leucotriène est un récepteur BLT.

**4.** Composition destinée à être utilisée dans le traitement ou la prévention des maladies citées selon la revendication 1, dans lequel le récepteur de leucotriène est un récepteur CysLT1.

**5.** Composition destinée à être utilisée dans le traitement ou la prévention des maladies citées selon l'une quelconque des revendications 1 à 4, dans lequel la composition est une composition alimentaire.

**6.** Composition destinée à être utilisée dans le traitement ou la prévention des maladies citées selon l'une quelconque des revendications 1 à 4, dans lequel la composition est une composition pharmaceutique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004047554 A
- US P3773919 A
- EP 58481 A
- EP 133988 A
- DE 3218121
- EP 52322 A
- EP 36676 A
- EP 88046 A
- EP 143949 A
- EP 142641 A
- JP 58118008 A
- US P4485045 A
- US P4544545 A
- EP 102324 A


### Non-patent literature cited in the description


- **TAGER AM ; LUSTER AD.** The Leukotriene B4 Receptors. *Prostaglandins Leukot Essent Fatty Acid.,* August 2003, vol. 69 (2-3), 123-34
- **CAPRA V ; AMBROSIO M ; RICCIONI G ; ROVATI GE.** Cysteinyl-Leukotriene receptor antagonists: Present Situation and Future Opportunities. *Curr Med Chem.,* 2006, vol. 13 (26), 3213-26
- **MASAYOSHI ABE ; TANIHIRO YOSHIMOTO.** Leukotriene Lipoxygenase: Metabolic System and Drug Discovery. *Folia Pharmacologica Japonica,* 2004, vol. 124, 415-425
- **SIDMAN et al.** *Biopolymers,* 1983, vol. 22, 547-556
- **LANGER et al.** *J. Biomed. Mater. Res.,* 1981, vol. 15, 167-277
- **LANGER.** *Chem. Tech.,* 1982, vol. 12, 98-105
- **LANGER.** *CHEM. TECH.*
- **LANGER.** *Science,* 1990, vol. 249, 1527-1533
- **TREAT et al.** Liposomes in the Therapy of Infectious Disease and Cancer. Liss, 1989, 317-327, 353-365
- **EPSTEIN et al.** *Proc. Natl. Acad. Sci. USA,* 1985, vol. 82, 3688-3692
- **HWANG et al.** *Proc. Natl. Acad. Sci. USA,* 1980, vol. 77, 4030-4034
- **SEFTON.** *CRC Crit. Ref. Biomed. Eng.,* 1987, vol. 14, 201
- **BUCHWALD et al.** *Surgery,* 1980, vol. 88, 507
- **SAUDEK et al.** *N. Engl. J. Med.,* 1989, vol. 321, 574